# EUROPEAN PATENT SPECIFICATION

(11) **EP 2 490 644 B1**
(45) Date of publication and mention of the grant of the patent: **03.09.2014**
(21) Application number: 09806202.9
(22) Date of filing: 29.12.2009
(51) Int. Cl.: A61H 7/00, A61H 19/00, A61N 5/06

(54) **AN APPARATUS FOR TREATING AND/OR PREVENTING DISEASES AND FUNCTIONAL DISORDERS OF EXTERNAL GENITAL ORGANS**
VORRICHTUNG ZUR BEHANDLUNG UND/ODER PRÄVENTION VON ERKRANKUNGEN UND FUNKTIONELLEN STÖRUNGEN DER EXTERNEN GESCHLECHTSORGANE
APPAREIL DE TRAITEMENT ET/OU PRÉVENTION DE MALADIES ET DE TROUBLES FONCTIONNELS DES ORGANES GÉNITAUX EXTERNES

(30) Priority: 12.10.2009 EA 200901468
(43) Date of publication of application: 29.08.2012
(73) Proprietor: Pletnev, Sergey Vladimirovich, 220000 Minsk (BY)
(72) Inventor: Pletnev, Sergey Vladimirovich, 220000 Minsk (BY)
(74) Representative: Anohins, Vladimirs
(86) International application number: PCT/IB2009/055987
(87) International publication number: WO 2011/045632

(56) References cited:
- WO-A1-98/25667
- CA-A1- 2 282 723
- RU-C1- 2 153 366
- US-A1- 2004 260 209
- US-A1- 2008 243 197
- US-B1- 6 443 978

## Description

The present invention is related to the field of medical equipment, specifically to means for treating and preventing diseases and functional disorders (stimulating) genital organs.

A number of methods and apparatuses has been proposed for treatment of impotence by exposing an organ to mechanical oscillations and a magnetic field (International Application WO2005041845).

The method and apparatus have been proposed for a combined action of vacuum and infrared light (AMVL-01) apparatus developed by "Yarovit-Yar Ltd".

The above methods are efficient for treatment and prevention of functional disorders and a number of diseases.

Such methods and apparatuses, however, are useful for treatment of a limited range of diseases.

A number of methods and apparatuses for treatment of impotence and scleroderma using the periodic exposure to vacuum and a travelling magnetic field has been proposed (RU Patent No. 2072822).

This method is also useful only for treatment of impotence and localized scleroderma.

From the prior art there is known WO 98/25667 A1 (ELECTROGESIC CORP [US]; PARRIS OANNY M [US]) 18 June 1998 (1998-06-18) that describes the generation of harmonious signals, e.g. notes and their harmonics and subharmonics. These signals can be further modulated according to different laws and then directed to different applicators selected from the group consisting of infrared emitters, magnetic inductors, oscillators and electrostimulators. WO 98/25667 A1 teaches that specified applicators can be applied to different parts of the body, however genitals are not mentioned here. It should be noted that the design of the applicators described in WO 98/25667 A1 are applicable for any lateral area on the human body, but they cannot be not used for the male genital organ, while the technical solution asserted in the present application cannot be used otherwise but only for the male genital organ.

The apparatus according to Patent of Canada CA 2 282 723 A1 (ORPIN JOHN A [CA]) 3 March 2001 (2001-03-03) is designed for stimulation and comprises 3 sources of energy - light, magnetic and mechanical, whereby the magnetic field is presented in the form of pulsating unipolar and alternating bipolar magnetic fields. Therefore, D3 differs from the proposed invention both in the nature of the magnetic field and in absence of the vacuum system. Furthermore, the aserted solution also differs in the area of exposure and unique design.

US application 2008/243197 A1 (BOVE THOMAS [US]) 2 October 2008 (2008-10-02) describes an apparatus for stimulation which comprises 3 sources of energy - light, magnetic and mechanical, whereby the magnetic field is presented in the form of pulsating unipolar and alternating bipolar magnetic fields. The solution asserted in US application 2008/243197 also provides the lateral area of exposure and has unique design of inductors not suitable for the treatment of male genitals.

There are several embodiments known from patent RU 2 153 366 C1 (ZHAROV VLADIMIR PAVLOVICH) 27 July 2000 (2000-07-27).

The magnetic field in this patent is presented in the form of isolated short pulses for the "realization of noncontact electric stimulation of the prostate gland due to induction of EMF in neuromuscular structures" (fig .4a pos. 2). This fact is also confirmed in the description of Fig. 4.: "Fig. 4 shows a more detailed diagram of the photomagnetic therapeutic module. In two-dimensional geometry solenoid 1 (Fig. 4a) upon passing pulsed current a pulsed magnetic field is formed, flux lines of which (shown in solid lines) pierce the prostate gland 2 and induce corresponding EMF therein, i.e. distant electrostimulation is therefore realized". In the second embodiment in Fig. 4b the unit is presented in the form of a belt with inductors covering a person's body in section of the prostate gland. These embodiments are also suitable only for lateral applications.

The photo-vacuum module design (Fig. 3) includes special volume for exposure of man's genitals to vacuum and to a source of light but it does not provide for magnetic pulsed exposure, which fact limits the therapeutic potential I respect of the depth and intensiveness of optical radiation penetration and its exposure only to of skin surface cells of the organ.

The most relevant to proposed solution is the US 2004/0260209 (ELLA SIMA ET AL) 23-12-2004 which discloses a long list of different types of energy that may be delivered in tandem; however, a coordinated application of a suction massage and a pulsed magnetic field is not obvious in view of a long list of different therapy modes defined in claim 106. The document describes only a synchronized delivery of LLLT.

It provides only limited capacities and effectiveness of treatment.

The objective of the present invention is to expand therapeutic capacities and improve efficiency. The invention is defined in the appended set of claims.

The objective of the present invention is achieved by providing a system for treatment and/or prevention of diseases and functional disorders of external genital organs, comprising a source of vacuum with a control device configured to control periodic action of vacuum, and an isolated chamber adapted to accommodate a to-be-exposed organ and means for exposure of an organ to a light field with combination of different wavelengths. The system additionally includes means for exposing the organ to a low-frequency pulsed magnetic field, including a source of a low-frequency pulsed magnetic field with an inductor generating a magnetic field in the place of the location of the organ to be subjected to exposure. Furthermore, the system comprises a control device for synchronizing and combining the pulsed magnetic field with
- the pulsed light exposure with prescribed color light combinations and sequence thereof, and with periodic vacuum oscillations.

It should be noted that means for exposing the organ to a low-frequency pulsed magnetic field (19) provides the amplitude of the pulsed magnetic field in the range of 3-50 mT.

This combined exposure produces synergistic effect which enhances therapy intensity, substantially expands the rage of curable diseases and also significantly increases prevention efficiency. Additional light exposure makes it possible to treat an expanded range of diseases, for example, skin, blood and other diseases. In addition, such an intensive and combined effect on blood circulating the organ during the procedure is similar to the blood treatment by exfusion. It is noteworthy that a portion of blood which is actually treated over the entire procedure in the effective volume due to erection, is then flows through a body and produces therapeutic effect on other organs, thereby substantially stimulating functioning of all organs and improving the body resistance to other diseases.

Therefore, a combined, multimode effect of the pulsed magnetic field, multifunctional optical radiation due to an extensive use of different wavelengths, light flux polarization and also vacuum pull provide three main principles of physiotherapy: synergism, complementarity and partial antagonism that allows not only the list of diseases to be extended, but also - which is the most important of all that - the treatment quality to be improved and treatment time to be reduced.

The invention is illustrated by the following example embodiment set forth in accompanying drawings in which:

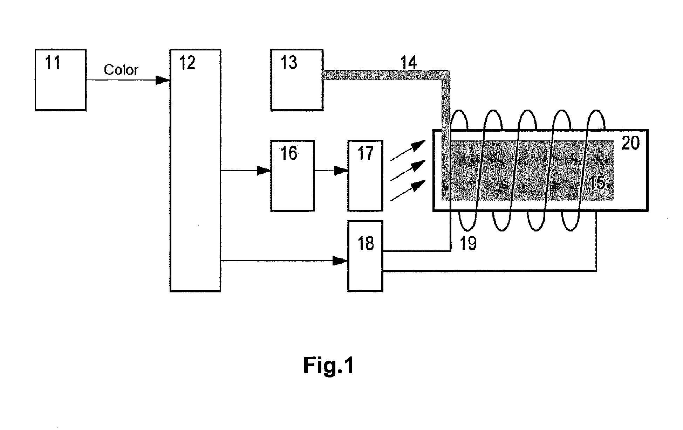

Fig. 1 - an embodiment of a block-diagram of the device with a combined vacuum, magnetic and light exposure.

It will be readily understood by those skilled in the art that these devices may be integrated.

Referring to Fig. 1, the device of the present invention comprises a control panel 11 coupled with a microcontroller 12 to enable and disable respective actions and combinations of the light exposure. A pneumatic bulb 13 is connected with a hose 14 and a pneumatic sleeve 15. Pressure in the system is controlled and set individually. A control unit 16 of a light-emitting diode array 17 is connected to the output of the microcontroller 12. A control unit 18 of an electromagnetic inductor 19 is connected to the second output of the microcontroller 12. The electromagnetic inductor 19 encloses an isolated flask 20 made of a light transmissive material. The pneumatic sleeve 15 is disposed on the inner surface of the flask 20 and is provided with elastic sealing.

The light-emitting diode array 17 is disposed outside in proximity to the light transmissive surface of the flask 20.

The principle of operation of the device is as describe hereinafter. A to-be-treated organ is inserted into the flask 20. Then the elastic sealing of the pneumatic sleeve 15 softly encloses it. The control panel 11 is used to set of color light combinations and sequence thereof. Pressure parameters are adjusted individually. Color Light combinations are program-set by guidelines of a physician or based on the sequential combination of the entire color set of the light-emitting diodes in the array. Frequency and intensity of magnetic field pulses may be also varied on physician's order or through the program based on the set standard sequences. The procedure time is also program-set or is stopped in case of onset of orgasm.

The vacuum exposure is selected by each patient individually with consideration for individual features, then the magnetic and phototherapeutic actions are synchronized, with the procedure duration being 10 minutes and increasing by 2 minutes each day reaching 20 minutes and then reducing down to 14 minutes. The treatment or prevention cycle reaches 10-12 days.

The magnetic field value may be varied within the range of 3-50 mT and is set on the average at 10 +/-5 mT. The light flux value is 2+/-1 mW.

The test conducted has demonstrated high efficiency of the claimed device. The device may be actually used for treatment of any diseases of organs in combination with the general exposure of organism to the above described factors.

The combined direct exposure of the organ to vacuum, low-frequency magnetic field and light in such a combination and geometry allows to achieve a new therapeutic effect, along with the advantages known before. At the same time the low-frequency pulsed magnetic field is formed in the form of pulse packets, e.g. as in (WO1999002217) MAGNETOTHERAPY DEVICE, (WO1999002218) DEVICE FOR LOCAL MAGNETOTHERAPY and can be used not only for electric pulsed stimulation, but for the treatment of tissues, fluids, cell membranes, blood cells and molecules. Vacuum exposure causes the afflux of blood to the organ and a passive erection, allowing to hold a significant portion of blood for photomagnetic treatment for a long time interval, this blood being activated in the process of such treatment then flows into the organism and causes activation of other organs, improves the immune resistance, as well as all provides other positive effects that could not be obtained before with the use of extracorporal blood treatment only (e.g. EA005256 (B1) - METHOD FOR CORRECTING OF HEMODYNAMICS AT SEPTIC SHOCK).

Therefore, in comparison with RU 2 153 366 C1 (ZHAROV) the proposed technical solution differs in the application area of the complex exposure comprising a magnetic field, in the nature of the magnetic field and the mechanism of its exposure and the object of exposure, i.e. it is novel and produces a new positive effect owing to the continious photomagnetic treatment of a significant blood portion, wherein the effectiveness of exposure, both magnetic and photonic, is significantly increased due to synergism and increase of the effective volume of exposure. Such complex exposure was not obvious, since it was not proposed by the inventor of RU 2 153 366 C1 (ZHAROV) or US 2004/0260209 (ELLA SIMA ET AL) and others, and the effect of the total exposure significantly exceeds the amount of effects of individual exposures. Furthermore, in this case the exposure is realized not only to the organ subjected to the therapy, but also the whole organism due to the accumulated exposure of the significant portion of blood, which then flows into the organism (this may be termed auto-hemo-photo-magnetotherapy, wherein a blood portion is continuously held in the organ due to the erection, treated with a magnetic field, vacuum and a laser radiation, and then returned into the organism).

## Claims

1. A system for treatment and/or prevention of diseases and functional disorders of external genital organs, comprising a light source (17) configured to provide a light field with a combination of different wavelengths, a source of vacuum (13) with a control device configured to control periodic action of vacuum, an isolated chamber (20) adapted to accommodate a genital organ and configured to expose the genital organ to said light field, further comprising a source of a low-frequency pulsed magnetic field (18) with an inductor (19) providing a pulsed magnetic field in the place of the location of the organ, and further comprising a device (12) configured to synchronize and combine the low-frequency pulsed magnetic field with a pulsed light exposure with prescribed color light combinations and sequence thereof ;
and configured to synchronize and combine the low-frequency
pulsed magnetic field with
periodic vacuum oscillations.

2. The system according to claim 1, **characterized in that** the inductor (19) provides an amplitude of the pulsed magnetic field in the range of 3-50 mT.

## Patentansprüche

1. System zur Behandlung und/oder Prävention von Erkrankungen und Funktionsstörungen der externen Geschlechtsorgane, mit einer Lichtquelle (17), konfiguriert zum Bereitstellen eines Lichtfelds mit einer Kombination von verschiedenen Wellenlängen, mit einer Vakuumquelle (13) mit einer Steuereinrichtung, konfiguriert zum Steuern einer periodischen Wirkung von Vakuum, einer zum Aufnehmen eines Genitalorgans isolierten Kammer (20), und konfiguriert ist, um das Genitalorgan zu dem Lichtfeld zu belichten, ferner umfassend eine Quelle eines niederfrequenzgepulsten Magnetfelds (18) mit einer Induktivität (19) bereitstellen ein gepulstes Magnetfeld in der Ort der Lage des Organs, und mit einer Vorrichtung (12), konfiguriert zum Synchronisieren und Kombinieren des niederfrequenzgepulsten Magnetfelds mit einer gepulsten Belichtung mit vorgegebenen Farblichtkombinationen und Abfolge davon; und zu Synchronisieren und Kombinieren des niederfrequenzgepulsten Magnetfelds mit periodischer Vakuumschwingungen konfiguriert ist.

2. System nach Anspruch 1, **dadurch gekennzeichnet, dass** der Induktor eine Amplitude des gepulsten Magnetfelds im Bereich von 3 bis 50 mT (19) liefert.

## Revendications

1. Système pour le traitement et/ou la prévention de maladies et de troubles fonctionnels des organes génitaux externes, comprenant une source de lumière (17) configuré de manière à fournir un champ lumineux à l'aide d'une combinaison de différentes longueurs d'onde, une source de vide (13) avec un dispositif de commande configuré pour commander une action périodique de vide, une chambre isolée (20) adapté pour recevoir un organe génital et configuré pour exposer l'organe génital pour ledit champ de lumière, comprenant en outre une source de champ magnétique pulsé basse fréquence (18) avec une bobine d'inductance (19) la fourniture d'un champ magnétique pulsé à la place de l'emplacement de l'organe, et comprenant en outre un dispositif (12) configuré pour synchroniser et de combiner le champ magnétique pulsé basse fréquence avec une lumière pulsée d'exposition avec une lumière de couleur prescrite des combinaisons et de la séquence de celui-ci; et configuré pour synchroniser et de combiner le champ magnétique pulsé basse fréquence avec des oscillations à vide périodique.

2. Système selon la revendication 1, **caractérisé en ce que** la bobine d'induction (19) fournit une amplitude de ce champ magnétique pulsé dans la plage de 3 à 50 mT.
